(19) Europäisches Patentamt
European Patent Office
Office européen des brevets

(11) EP 3 745 411 A1

(12) EUROPEAN PATENT APPLICATION

(43) Date of publication:
02.12.2020 Bulletin 2020/49

(51) Int Cl.:
G16H 50/20 (2018.01)　　　G16H 50/70 (2018.01)

(21) Application number: 19382423.2

(22) Date of filing: 27.05.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Universitat Pompeu Fabra
08002 Barcelona (ES)

(72) Inventors:
• VILA VIDAL, Manel
08005 Barcelona (ES)
• TAUSTE CAMPO, Adrià
08750 Molins de Rei (ES)

(74) Representative: Juncosa Miró, Jaime
Torner, Juncosa i Associats, S.L.
Gran Via de les Corts
Catalanes, 669 bis, 1o, 2a
08013 Barcelona (ES)

(54) **A COMPUTER IMPLEMENTED METHOD AND COMPUTER PROGRAM PRODUCTS FOR IDENTIFYING TIME-FREQUENCY FEATURES OF PHYSIOLOGICAL EVENTS**

(57)　A computer implemented method and computer programs for identifying time-frequency features of physiological events are disclosed. The method comprises selecting, by a computing system, some of a set of physiological signals associated with a physiological event, referred as targeted physiological signals, and filtering the targeted signals within defined time-frequency windows; calculating, for each defined time-frequency window, a given feature of each targeted signal limited within the time-frequency window, providing for each window a sample of the feature over all targeted physiological signals; and calculating, for each feature sample, a first quantifier defined directly over the feature sample and comparing the calculated first quantifier with a given threshold, the computing system further selecting the time-frequency windows satisfying the threshold; and/or a second quantifier defined over an empirical distribution of the feature sample and comparing the calculated second quantifier with a given threshold, the computing system further selecting the time-frequency windows satisfying the threshold.

Fig. 1

## Description

Technical Field

The present invention relates to a computer implemented method and to computer program products for identifying the characteristic time-frequency features of physiological events. In particular the invention can be used to extract the spectral features of the electrophysiological seizure onset patterns and to predict the epileptic focus.

Background of the Invention

In patients with drug-resistant epilepsy, the success of the resective surgery depends critically on the accurate definition of the epileptogenic zone (EZ). In particularly challenging cases, the use of invasive recording techniques to monitor intracranial electroencephalography (iEEG) activity might be required during the pre-surgical evaluation to determine the cortical areas to be resected.

Over the last decades, an increasing effort has been undertaken to develop quantitative tools for iEEG analysis to better characterize and understand how epileptic seizures are generated and propagated, a complex problem that involves both temporal and spatial variables. On the temporal domain, for instance, several studies have aimed to design methods that can efficiently and automatically detect interictal spikes high-frequency oscillations, or that can even predict seizures. In contrast, the development of automated methods to delineate the spatial localization of the seizure-onset zone (SOZ) remains challenging due to a number of reasons. The complex localization of the SOZ, the variable number and typology of seizures during the monitoring period and the variety of electrophysiological seizure-onset patterns that may occur even within one patient represent serious challenges to design a detection algorithm that is universally valid for all patients.

While the gold standard in SOZ identification is still retrospective visual inspection of iEEG recordings, several biomarkers have been proposed to characterize the epileptogenicity of the monitored brain structures (Bartolomei et al., 2008; David et al., 2011; Gnatovsky et al., 2011, 2014; Andrzejak et al., 2015; Vila-Vidal et al., 2017). These biomarkers quantify preselected spectral features of the iEEG signals in order to assess the degree of involvement of each region in the seizure process. Although the SOZ might be indirectly delineated based on the epileptogenicity of each brain structure, none of the cited studies explicitly aims to design or implement fully unsupervised algorithms. More recent studies have proposed automatic methods based on high-frequency oscillations (HFOs) in interictal or peri-ictal periods, or stochastic properties of the iEEG signals in predefined frequency windows (Geertsema et al. 2015; Liu et al., 2016; Murphy et al., 2017; Varatharajah et al.,

2017).

There are also known some patents or patent applications in this field.

US 9326698-B2 discloses a method that detects oscillatory signals representative of discrete events in a patient's body. The detected signals may be tested in the context of surrounding background activity to identify anomalous discrete physiological events that are sufficiently different from the surrounding background activity. The anomalous discrete physiological events having correlative morphological, time, or location characteristics may be automatically clustered and clusters of anomalous physiological events may be determined that are indicative of at least one region of the patient's body that is associated with a medical condition.

US 6678548-B1 discloses a method for predicting and detecting epileptic seizure onsets within a unified multiresolution probabilistic framework, enabling a portion of the device to automatically deliver a progression of multiple therapies, ranging from benign to aggressive as the probabilities of seizure warrant. Based on novel computational intelligence algorithms, a realistic posterior probability function $P(St|x)$ representing the probability of one or more seizures starting within the next T minutes, given observations x derived from iEEG or other signals, is periodically synthesized for a plurality of prediction time horizons. When coupled with optimally determined thresholds for alarm or therapy activation, probabilities defined in this manner provide anticipatory time-localization of events in a synergistic logarithmic-like array of time resolutions, thus effectively circumventing the performance vs. prediction-horizon tradeoff of single-resolution systems. The longer and shorter prediction time scales are made to correspond to benign and aggressive therapies respectively. The imminence of seizure events serves to modulate the dosage and other parameters of treatment during open-loop or feedback control of seizures once activation is triggered. Fast seizure onset detection is unified within the framework as a degenerate form of prediction at the shortest, or even negative, time horizon.

Other methods are known by US 2016045127-A1, US 2016287118-A1, WO 2018005981-A1 and US 6594524-B2.

In most of the aforementioned studies or patents, the proposed biomarkers are critically built around spectral features that are confined to predefined frequency bands (either high frequency or whole-spectrum) and might fall short to capture patient-specific seizure onset patterns. Yet, there is no adaptive algorithm that extracts the most relevant features for SOZ localization before proceeding to the localization itself.

Hence, and in a more general fashion, there is a need to develop methodological tools that are capable of extracting the time-frequency features of physiological signals associated with the occurrence of a pre-defined physiological event. These tools could then be used to determine the brain sites underlying a pathological or a cognitive phenomenon in a patient- and event-specific context.

References

Andrzejak RG, David O, Gnatkovsky V, Wendling F, Bartolomei F, Francione S, et al. Localization of epileptogenic zone on pre-surgical intracranial EEG recordings: toward a validation of quantitative signal analysis approaches. Brain Topography 2015; 28(6): 832-837.

Bartolomei F, Chauvel P, Wendling F. Epileptogenicity of brain structures in human temporal lobe epilepsy: a quantified study from intracerebral EEG. Brain 2008; 131 (7): 1818-1830.

David O, Blauwblomme T, Job AS, Chabardès S, Hoffmann D, Minotti L, et al. Imaging the seizure onset zone with stereo-electroencephalography. Brain 2011; 134(10): 2898-2911.

Geertsema EE, Visser GH, Velis DN, Claus SP, Zijlmans M, Kalitzin SN. Automated seizure onset zone approximation based on nonharmonic high-frequency oscillations in human interictal intracranial eegs. International Journal of Neural Systems 2015, 25(05): 1550015.

Gnatkovsky V, Francione S, Cardinale F, Mai R, Tassi L, Lo Russo G, et al. Identification of reproducible ictal patterns based on quantified frequency analysis of intracranial EEG signals. Epilepsia 2011; 52(3): 477-488.

Gnatkovsky V, de Curtis M, Pastori C, Cardinale F, Lo Russo G, Mai R, et al. Biomarkers of epileptogenic zone defined by quantified stereo-EEG analysis. Epilepsia 2014; 55(2): 296-305.

Liu S, Sha Z, Sencer A, Aydoseli A, Bebek N, Abosch A, et al. Exploring the time-frequency content of high frequency oscillations for automated identification of seizure onset zone in epilepsy. Journal of Neural Engineering 2016; 13(2): 026026.

Murphy PM, von Paternos AJ, Santaniello S. A novel HFO-based method for unsupervised localization of the seizure onset zone in drug-resistant epilepsy. In: Engineering in Medicine and Biology Society (EMBC), 2017 39th Annual International Conference of the IEEE. IEEE, 2017. P. 1054-1057.

Varatharajah Y, Berry BM, Kalbarczyk ZT, Brinkmann BH, Worrell GA, and Iyer RK. Inter-ictal seizure onset zone localization using unsupervised clustering and bayesian filtering. In: Neural Engineering (NER), 2017 8th International IEEE/EMBS Conference on. IEEE, 2017. P. 533-539.

Vila-Vidal M, Principe A, Ley M, Deco G, Tauste Campo A, Rocamora R. Detection of recurrent activation patterns across focal seizures: Application to seizure onset zone identification. Clinical Neurophysiology 2017; 128(6): 977-985.

Description of the Invention

To that end, embodiments of the present invention provide, according to a first aspect, a computer implemented method for identifying time-frequency features of physiological events. The proposed method comprises receiving, by a computing system having at least one memory and one or more processors, a set of physiological signals associated with a physiological event and acquired from at least one sensor located in or on at least one recording site of a living being either a human or an animal; a time period in which the physiological event occurred; a time-frequency region of interest; and a plurality of time-frequency windows defined on the time-frequency region of interest.

The cited time-frequency region is defined by a minimum and a maximum time instant and a minimum and a maximum frequency, wherein said minimum and maximum time instants are comprised within said time period in which the physiological event occurred and said maximum frequency is lower than or equal to the Nyquist frequency of the physiological signals.

Once received the above data, the computing system may select some or all of the physiological signals, referred as targeted physiological signals, and filter the targeted physiological signals within said time-frequency windows. Then, the computing system may calculate, for each defined time-frequency window, a given feature of each one of the targeted physiological signals limited within the time-frequency window, providing for each time-frequency window a sample of the feature over all targeted physiological signals. For each feature sample, the computing system then may also calculate a first quantifier defined directly over the feature sample and/or a second quantifier defined over an empirical distribution of the feature sample.

If the first quantifier is calculated, the first quantifier is then compared with a given first threshold. In this case, the computer system may select the time-frequency windows satisfying the first threshold (i.e. being either above or below the threshold). If the second quantifier is calculated, this second quantifier is compared with a given second threshold. The computing system then may select the time-frequency windows satisfying the second threshold. Particularly, the first and second thresholds are different.

According to the present invention, the physiological event may be an epileptic seizure, the pre-ictal preparation phase, the brain response to a delivered cognitive or electrical stimulus, the brain response to the administration of a drug, etc. The physiological signals may particularly comprise intracranial electroencephalography (iEEG) signals.

According to an embodiment, the cited feature is an intrinsic attribute of the targeted physiological signal. The intrinsic attribute particularly includes the mean activation (MA), which is defined as the time-average normalized power in band (PIB) of the targeted physiological signal within the time-frequency window, the normalization being performed with respect to a pre-defined baseline distribution. The PIB can be computed using the Hilbert transform, the wavelet transform, the Fourier transform, etc.

In another embodiment, the cited feature is an attribute that indicates how a targeted physiological signal is related with respect to the other targeted physiological signals limited within the time-frequency region. For example, the attribute can be computed using a correlation strength measure, a betweenness centrality measure, a node degree measure, among others.

The first quantifier may comprise any of the mean, the standard deviation, the maximum or the global activation (GA) of the feature sample, etc. The second quantifier may comprise the Renyi entropy, the Fisher information or the activation entropy (AE), etc.

If the AE is calculated, the computer system may further compare the feature sample with a given third threshold for each time-frequency window satisfying the second threshold, thus defining, for each time-frequency window satisfying the second threshold, a subset of the targeted physiological signals, called relevant physiological signals; and accumulate all relevant physiological signals across time-frequency windows satisfying the second threshold.

According to the proposed method, the time-frequency windows may overlap or not with each other. Likewise, the time-frequency windows may have an equal or different width. Even, the time-frequency windows may be nested windows with initial bound fixed at said minimum time instant and with increasing final bound.

For a particular embodiment, the recorded signals are intracranial electroencephalography (iEEG) signals, the physiological event is an epileptic seizure, the cited feature is the mean activation (MA), the first and second quantifiers are the global activation (GA) and the activation entropy (AE), respectively, and the first and second thresholds are a lower threshold for GA and an upper threshold for AE, respectively. In this case, both quantifiers are computed and the computer system further comprises identifying the relevant physiological signals as described above. As a result, the time-frequency windows satisfying both the first and the second thresholds yield maximal and spatially confined spectral activations, thus ensuring that propagation has not started and that SOZ contacts can be naturally discriminated from other sites. The relevant physiological signals identify the seizure focus.

Other embodiments of the invention provide, according to other aspects, a system and computer program products including instructions embodied in a non-transitory computer readable medium that, when executed by a processor, cause the processor to identify time-frequency features of physiological events.

Hence, the embodiments described herein provide a fully unsupervised and automatic methodology for identifying time-frequency features of physiological events, such as the epileptic seizure onset, epileptogenic sites responding to electrical stimulation, brain sites that respond to drug administration, brain sites that respond to any kind of cognitive stimulation during a task paradigm, etc.

The proposed method carries minimal computational costs. Although the method relies on the a priori detection of the physiological event (e.g. the seizure onset time defined by the clinical neurophysiologist), it needs no information about the frequency and temporal windows of interest, two parameters that are automatically extracted from the spectral properties of the signal. Finally, and more importantly, the achieved automatization of the method does not come at the expense of interpretability. The output of the analysis (the time-frequency windows and the SOZ) can easily be understood as describing the spectral properties (characteristic frequency, duration and spatial localization) of an electrophysiological pattern during an event of interest. Overall, the proposed procedure is particularly suitable to be used as a complementary tool during the pre-surgical evaluation and planning that might help better identify and interpret the regions involved in seizure generation and propagation.

Brief Description of the Drawings

The previous and other advantages and features will be more fully understood from the following detailed description of embodiments, with reference to the attached figures, which must be considered in an illustrative and non-limiting manner, in which:

    Fig. 1 is a flow chart illustrating a method for identifying time-frequency features of physiological events according to an embodiment of the present invention.

    Fig. 2 is an exploration of spectral activations in different time-frequency windows of interest done around seizure onset.

    Fig. 3 illustrates the processing steps from iEEG signals to SOZ detection.

Detailed Description of Preferred Embodiments

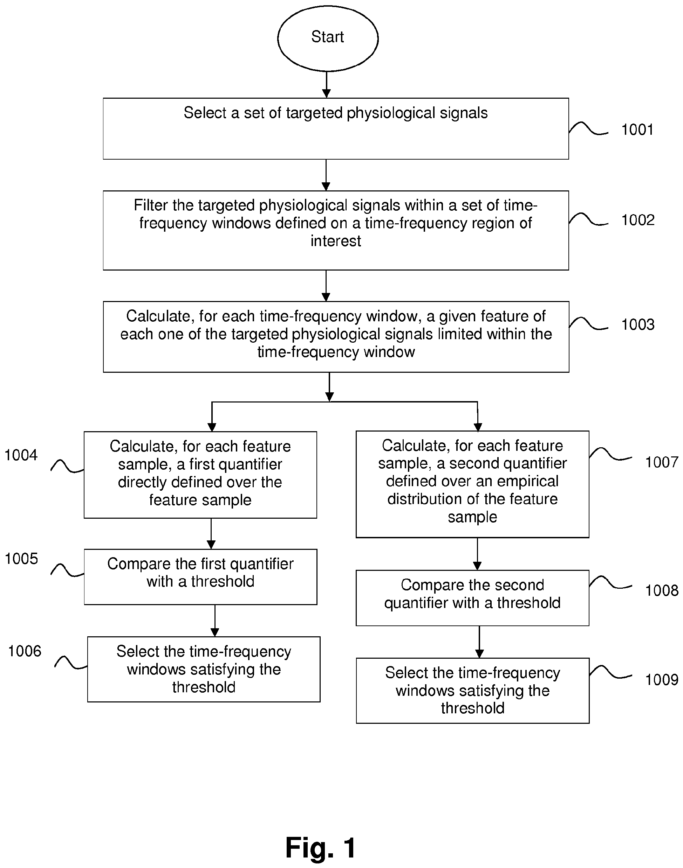

Fig. 1 illustrates a general overview of a method for identifying time-frequency features of physiological events. The method is implemented/executed by a computer and is applied to a set of physiological signals associated with a physiological event and acquired/recorded from different recording sites. The physiological signals can be acquired from a human or an animal.

The general purpose of the method is: 1) to identify the characteristic time and frequency scale of spectral changes in said physiological signals associated with (preceding, following and/or co-occurring with) a particular event for which the time boundaries are (approximately) known; and 2) in the case that the event of interest is spatially confined, to determine its spatial localisation, i.e., the region where it occurred. In a particular embodiment as will be detailed later, 1) to identify the characteristic time and frequency scale of spectral changes in electrical signals from the brain that occur at seizure onset and 2) when seizure onset is spatially confined, to identify the seizure onset zone (SOZ).

To that end, besides the physiological signals, as input to the method there is also needed a time period in which the physiological event occurred; a time-frequency region of interest defined by a minimum and a maximum time instant comprised within the time period in which the physiological event occurred and a minimum and a maximum frequency, the latter being lower than or equal to the Nyquist frequency of the physiological signals; and a plurality of time-frequency windows defined on the time-frequency region of interest.

It should be noted that the plurality of time-frequency windows may overlap or not with each other and may have equal or unequal widths. Moreover, in some cases it might be preferable to use integrating time-frequency windows to quantify the accumulation of activity, i.e. nested windows with initial bound fixed at the cited minimum time instant and with increasing final bound.

Referring back to Fig. 1, at step 1001, the computer selects at least some of the received physiological signals, referred as targeted physiological signals, and filters, step 1002, the targeted physiological signals within the time-frequency windows. At step 1003, the computer calculates, for each defined time-frequency window, a given feature of each one of the targeted physiological signals limited within the time-frequency window, providing for each time-frequency window a sample of the feature over all targeted physiological signals. For example, in an embodiment, the given feature is an intrinsic attribute of the targeted physiological signal including the mean activation (MA) defined as the time-average normalized power in band (PIB) of the targeted physiological signal within the time-frequency window. The PIB can be computed using the Hilbert transform method in narrow bands, but other methods such as the wavelet transform or the Fourier transform could be also used. In another embodiment, the given feature is an attribute that indicates how related a targeted physiological signal is with regard to the other targeted physiological signals limited within the time-frequency region, for example measures such as network centrality built upon within-frequency couplings or cross-frequency coupling can be used.

Then, the computer for each feature sample may calculate a first quantifier defined directly over the feature sample (step 1004) such as the mean, the standard deviation, the maximum or the global activation (GA), that quantifies the highest activations in the time-frequency windows, or may calculate a second quantifier defined over an empirical distribution of the feature sample (step 1007) such as the Fisher information, the Renyi entropy or the activation entropy (AE), that quantifies the structure or confinement or predictability of the feature sample in the time-frequency window. It should be noted that in other embodiments both first and second quantifiers may be calculated (i.e. both steps 1004 and 1007 may be executed).

After step 1004, the calculated first quantifier is compared, step 1005, with a first threshold. Finally, at step 1006, the computer selects only those time-frequency windows satisfying the threshold. The same applies for the second quantifier, as shown at steps 1008 and 1009.

Following, a particular embodiment of the proposed method for seizure onset pattern identification and epileptic focus prediction will be detailed. This is of particular importance in patients with drug-resistant epilepsy, as the accurate localization of the SOZ is crucial to plan a successful surgery.

To test the method, ten patients with pharmacoresistant epilepsy that underwent stereotactic-EEG in Hospital del Mar, Barcelona, were selected. Patient inclusion was based on the following criteria: a) that the seizure focus had been identified by the epileptologists and b) that ictal onset was confined to a reduced number of contacts. After electrode implantation and monitoring, SOZ was identified by neurologists using visual inspection. Surgical resection or radio-frequency thermocoagulation (RFTC) was planned based on individual SEEG evaluations.

Intracranial EEG recordings were obtained using 5 to 21 intracerebral multiple contact electrodes that were stereotactically implanted using robotic guidance. Signals were recorded using a standard clinical EEG system with 500 Hz of sampling rate, except for patient 3, where a sampling rate of 250 Hz was used. Intracranial EEG recordings from a total of 67 spontaneous seizures were collected and analyzed. Seizure onset and termination times of each seizure were independently marked by two epileptologists. For each seizure the computer selected the marked ictal epoch together with 60 seconds of pre-ictal baseline activity. Artifacted channels were identified by visual inspection and removed prior to data analysis. A band-pass filter (FIR, filter band [1,165] Hz) was used to remove slow drifts and aliasing effects. A notch FIR filter at 50 Hz and its harmonic frequencies was also used to remove the alternate current interference.

Then, information about the SOZ was extracted using the global activation (GA) and the activation entropy (AE) in an epoch defined around the ictal event (peri-ictal period). The current approach builds upon the previously introduced mean activation (MA) measure (Vila-Vidal *et al.*, 2017), which quantifies the average spectral activation of each targeted brain structure with respect to a pre-ictal baseline (from 60 to 20 seconds before ictal onset) for pre-defined frequency and time windows of interest. The MA of a given region j in the frequency band f and computed over a time-frequency window spanning from the seizure onset until time $t$ will be denoted with the following notation: $MA_j(f, t)$.

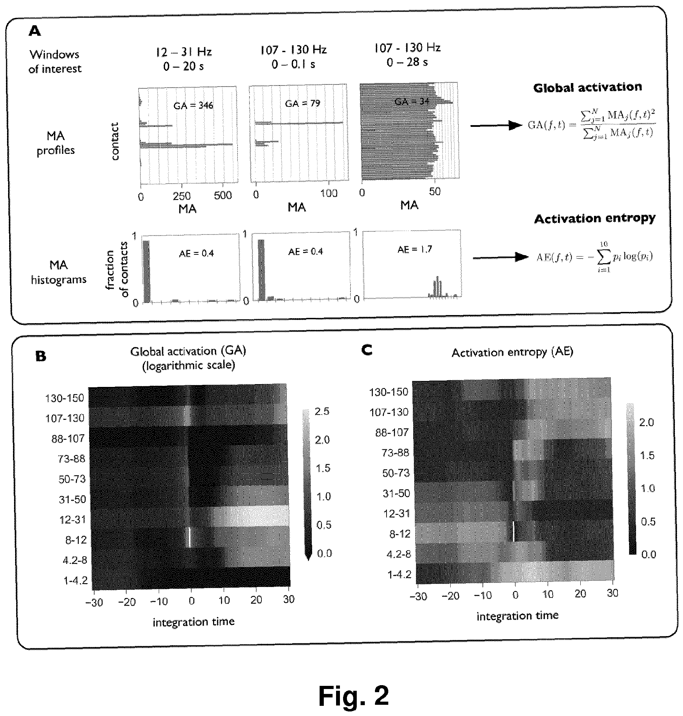

For optimal focus detection it must be ensured that there is a hierarchical and selective activation of SOZ contacts only. Central to the proposed approach is the definition of two novel measures, namely the GA and the AE, that in this particular case are jointly optimized to find time-frequency windows of interest where ictal activity is maximal with respect to a baseline pre-ictal period, while being spatially confined to a few contacts. Fig. 2 illustrates how these measures are computed and used to assess the amount of information carried in each window of interest. On one hand, the GA quantifies the magnitude of the most relevant spectral activations with respect to the pre-ictal basal state for a given time-frequency window of interest. It is defined as the weighted average MA over all contacts, where each contact's contribution is weighted by its own activation, thus ensuring that most active regions have a higher impact on the final value (Fig. 2A):

$$GA(f,t) = \frac{\sum_{j=1}^{N} w_j * MA_j(f,t)}{\sum_{j=1}^{N} w_j},$$

with $w_j = MA_j(f,t)$ if $MA_j(f,t) > 0$, and $w_j = 0$, if $MA_j(f,t) \leq 0$.

On the other hand, the AE is defined as the entropy of the MA distribution and characterizes the spatial spread of spectral activations for the given time-frequency of interest. First, the MA histogram is computed using 10 bins homogeneously spaced between the minimum and maximum MA values. Probability values for each bin ($p_i$ for $i = 1, ...,10$) are found as the fraction of contacts lying within the corresponding MA bin (Fig. 2A). The computer then computes the Shannon's entropy of this distribution using the formula:

$$GA(f,t) = -\sum_{i=1}^{10} p_i * \log p_i.$$

In order to systematically explore different windows for optimal SOZ detection the computer first divided the frequency spectrum into 10 non-overlapping bands using the following cutting points: 1, 4.2, 8, 12, 31, 50, 73, 88, 107, 130 and 150 Hz. In each band a set of 75 different integration time windows obtained by fixing the initial bound at seizure onset and varying the final bound from 0.1 to 30 s (in steps of 0.1 s in the range 0.1-5 s and steps of 1 s in the range 5-30 s) were considered. For each time-frequency window, the computer computed the MA profile and extracted its GA and AE. Fig. 2B and 2C show the GA and AE distributions for one exemplary seizure of patient 1, respectively.

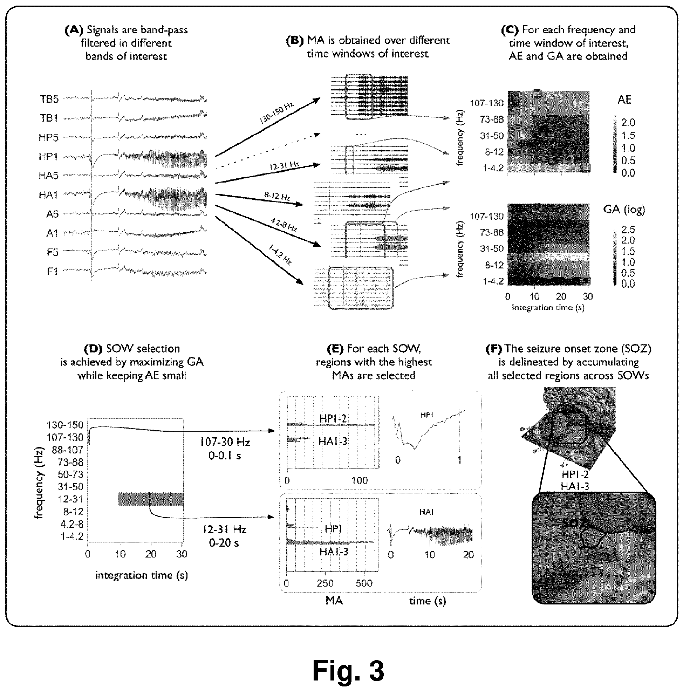

The procedure described before was sequentially applied to the 67 seizures included in this study. Fig. 3 summarizes the processing steps. Intracranial EEG signals in the peri-ictal period were band-pass filtered in pre-defined bands of interest spanning the whole spectrum (Fig. 3A). Then, for each band, MAs were obtained for all possible time windows of interest (Fig. 3B). For each time-frequency window, the computer extracted the GA and AE from the MA distribution (Fig. 3C).

Seizure onset window (SOW) detection was achieved by finding time-frequency windows that maximized the GA under the constraint of low AE to ensure that spectral activations were confined only to a few contacts. All pairs (f, t) were considered by the computer and two threshold conditions, one per variable, were set. For each seizure, a first threshold was set at the 95-th percentile of the GA distribution. In any case, the first threshold was always kept larger than 3 to ensure significant global activations with respect to the pre-ictal state. On the other hand, a second threshold of 0.5 was set on the AE, a condition that mathematically requires at least 80% of the contacts to lie within the same MA bin. Specifically, they were required to lie within the lowest MA bin. All time-frequency windows satisfying both conditions were preselected as candidates to be SOWs. Finally, for each frequency band the first time windows satisfying the condition were only kept. Fig. 3D shows the selected SOWs for the first seizure of patient 1.

In each seizure, the SOWs were qualitatively found to pinpoint the characteristic frequency and time windows of the seizure onset patterns. As an example, in the first seizure of patient 1, the algorithm selected the following SOWs: 107-130 Hz during the first hundreds of milliseconds and 12-31 Hz between 10 and 20 s. Regions of the posterior and anterior hippocampi were selected as being inside the SOZ. The inspection of the electrophysiological activity around the seizure onset by epileptologists revealed that the output of the method was qualitatively describing the seizure onset patterns. As seen in Fig. 3E the seizure is initiated at a hippocampal level with rapid discharges (~110 Hz) of very low amplitude in the first hundreds of milliseconds after seizure onset, combined with an inverted depolarizing wave. These discharges are particularly clear in HP1 and are followed by a drastic decrease in frequency evolving to low-voltage fast-activity (LVFA) at 12-31 Hz that becomes visible 5 seconds after seizure onset and that increases in amplitude as the seizure progresses. In this case, activations identified at 12-31 Hz are in fact a combined effect of LVFA activity (~30 Hz) together with slow rhythmic spikes (RS) (~15 Hz) of high amplitude particularly observable between 10 and 20 s.

In each SOW, the relevant iEEG channels were found using the property that at least 80% of the contacts are confined to the first bin of the MA histogram, a property that automatically induces a third threshold on the MA profile. The few remaining active contacts were considered to be part of the SOZ. This procedure was repeated for all selected SOWs, and SOZ contacts were accumulated, thus obtaining a single SOZ per seizure (Fig. 3F).

For each patient, the SOZ was defined by accumulating all seizure-specific detected SOZ regions. Then the SOZ defined by epileptologists was used as the ground truth to assess the performance of the proposed method. For each patient, the sensitivity and specificity of the selection were computed. The average sensitivity of the method across patients was $0.94 \pm 0.09$ (mean $\pm$ standard deviation), with an average specificity of $0.90 \pm 0.09$ (mean $\pm$ standard deviation). In 7 patients all SOZ regions were identified by the method (sensitivity=1). In the remaining 3 false negative cases (SOZ contacts mistakenly marked as non-SOZ) lied at most 1 contact (i.e. 1.5 mm) apart from true positives (regions correctly marked asSOZ).

The robustness of the method against particular choices of the thresholds was studied by computing a ROC curve as the thresholds varied. Small fluctuations of the GA threshold (95th percentile) and the AE threshold (0.5) were not found to significantly alter the sensitivity and specificity of the results. As the conditions on GA and AE are relaxed more non-SOZ sites are chosen. Despite being outside the SOZ, it was hypothesized that these sites might have a critical role in sustaining and propagating epileptic activity in the early stages of the seizure.

The amount of SOZ predictability carried in the pre-ictal activity was also assessed. To do so, time windows spanning from seizure onset into the past, i.e., with final bound at seizure onset and initial bound ranging from 30 s to 0.1 s before seizure onset, with the same spacing as before, were also considered. The average sensitivities and specificities of the method across patients in the pre-ictal period were lower than in the ictal period: $0.77 \pm 0.32$ (mean $\pm$ standard deviation) and $0.77 \pm 0.12$, respectively. Although the method has a better performance in the ictal period, high sensitivities and specificities indicate that the pre-ictal period contains sufficient information for SOZ prediction.

Finally, to relate the results of the proposed method with validated post-operative information, patients that underwent either surgery (n=5) or RFTC (n=4) were sub-selected and the degree of overlap between the predicted SOZ and the treated areas was quantified. Cross-validation with postoperative outcome showed that the fraction of predicted SOZ regions that were treated tended to be higher in patients attaining seizure freedom after a sufficiently long follow-up period that in those exhibiting symptom relapse after the treatment, being this trend higher in surgery than in RFTC. Yet, the proportion of treated SOZ regions lies in the range 0.25-0.85, highlighting the non-trivial relationship between the SOZ and the epileptogenic zone.

The present invention has been described in particular detail with respect to specific possible embodiments. Those of skill in the art will appreciate that the invention may be practiced in other embodiments. Further, the system and/or functionality of the invention may be implemented via various combinations of software and hardware, as described, or entirely in software elements. Also, particular divisions of functionality between the various components described herein are merely exemplary, and not mandatory or significant. Consequently, functions performed by a single component may, in other embodiments, be performed by multiple components, and functions performed by multiple components may, in other embodiments, be performed by a single component.

Certain aspects of the present invention include process steps or operations and instructions described herein in an algorithmic and/or algorithmic-like form. It should be noted that the process steps and/or operations and instructions of the present invention can be embodied in software, firmware, and/or hardware, and when embodied in software, can be downloaded to reside on and be operated from different platforms used by real-time network operating systems.

The scope of the present invention is defined in the following set of claims.

## Claims

1. A computer implemented method for identifying time-frequency features of physiological events, the method comprising:

   a) receiving, by a computing system:

      a set of physiological signals associated with a physiological event and acquired from at least one sensor located in or on at least one recording site of a living being;
      a time period in which the physiological event occurred;
      a time-frequency region of interest, said time-frequency region being defined by a minimum and a maximum time instant and a minimum and a maximum frequency, wherein said minimum and maximum time instants are comprised within said time period in which the physiological event occurred and said maximum frequency is lower than or equal to the Nyquist frequency of the physiological signals; and
      a plurality of time-frequency windows defined on the time-frequency region of interest;

   b) selecting, by the computing system, at least some of said physiological signals, referred as targeted physiological signals, and filtering the targeted physiological signals within said time-frequency windows;

c) calculating, by the computing system, for each defined time-frequency window, a given feature of each one of the targeted physiological signals limited within the time-frequency window, providing for each time-frequency window a sample of the feature over all targeted physiological signals; and

d) calculating, by the computing system, for each feature sample, at least one of:

a first quantifier defined directly over the feature sample and comparing the calculated first quantifier with a given first threshold, the computing system further selecting the time-frequency windows satisfying the first threshold; and/or

a second quantifier defined over an empirical distribution of the feature sample and comparing the calculated second quantifier with a given second threshold, the computing system further selecting the time-frequency windows satisfying the second threshold.

2. The method of claim 1, wherein said given feature calculated in step d) is an intrinsic attribute of the targeted physiological signal, the intrinsic attribute including the mean activation, MA, defined as the time-average normalized power in band, PIB, of the targeted physiological signal within the time-frequency window, the normalization being performed with respect to a pre-defined baseline distribution.

3. The method of claim 1, wherein said given feature in step d) is an attribute that indicates how a targeted physiological signal is related with respect to the other targeted physiological signals limited within the time-frequency region.

4. The method of any of previous claims, wherein the first quantifier comprises at least one of the mean, the standard deviation, the maximum or the global activation, GA, of the feature sample.

5. The method of any of previous claims, wherein the second quantifier comprises at least one of the Renyi entropy, the Fisher information or the activation entropy, AE, of an empirical distribution of the feature sample.

6. The method of any of previous claims 1 to 3, wherein the first quantifier comprises the global activation, GA, of the feature sample and wherein the second quantifier comprises the activation entropy, AE.

7. The method of claim 6, further comprising:

comparing the feature sample with a given third threshold for each time-frequency window satisfying the second threshold, thus defining, for each time-frequency window satisfying the second threshold, a subset of the targeted physiological signals, called relevant physiological signals; and

accumulating all relevant physiological signals across time-frequency windows satisfying the second threshold.

8. The method of any of previous claims, wherein the plurality of time-frequency windows overlap with each other.

9. The method of any of previous claims 1 to 6, wherein the plurality of time-frequency windows do not overlap with each other.

10. The method of any of previous claims, wherein the plurality of time-frequency windows have an equal width.

11. The method of any of previous claims 1 to 9, wherein the plurality of time-frequency windows have a different width.

12. The method of any of previous claims, wherein the plurality of time-frequency windows are nested windows with initial bound fixed at said minimum time instant and with increasing final bound.

13. The method of any of previous claims, wherein the physiological event is an epileptic seizure, the brain response to a delivered stimulus or the brain response to the administration of a drug, and wherein the physiological signals are intracranial electroencephalography, iEEG, signals.

14. A computer readable medium containing a computer program product for identifying time-frequency features of physiological events, the computer program product comprising program instructions that based on:

a set of physiological signals associated with a physiological event and acquired from at least one sensor located in or on at least one recording site of a living being;

a time period in which the physiological event occurred;

a time-frequency region of interest, said time-frequency region being defined by a minimum and a maximum time instant and a minimum and a maximum frequency, wherein said minimum and maximum time instants are comprised within said time period in which the physiological event occurred and said maximum frequency is lower than or equal to the Nyquist frequency of the physiological signals; and
a plurality of time-frequency windows defined on the time-frequency region of interest:

- select at least some of said physiological signals, referred as targeted physiological signals, and filtering the targeted physiological signals within said time-frequency windows;
- calculate, for each defined time-frequency window, a given feature of each one of the targeted physiological signals limited within the time-frequency window, providing for each time-frequency window a sample of the feature over all targeted physiological signals; and
- calculate, for each feature sample, at least one of:

a first quantifier defined directly over the feature sample and comparing the calculated first quantifier with a given first threshold, the computing system further selecting the time-frequency windows satisfying the first threshold; and/or

a second quantifier defined over an empirical distribution of the feature sample and comparing the calculated second quantifier with to a given second threshold, the computing system further selecting the time-frequency windows satisfying the second threshold.

Fig. 1

Fig. 2

**Fig. 3**

Europäisches
Patentamt

European
Patent Office

Office européen
des brevets

# EUROPEAN SEARCH REPORT

Application Number

EP 19 38 2423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/245481 A1 (BLANCO JUSTIN [US] ET AL) 27 September 2012 (2012-09-27) * abstract * * paragraphs [0010], [0038], [0040], [0085], [0115], [0152], [0216], [0228], [0231], [0254]; claim 1; figure 13 * | 1-14 | INV. G16H50/20 ADD. G16H50/70 |
| A | US 2007/250133 A1 (CARLSON DAVID L [US] ET AL) 25 October 2007 (2007-10-25) * abstract * * paragraph [0062] * | 1-14 | |
| A | WO 2016/200952 A1 (CHILDREN'S MEDICAL CENTER CORP [US]) 15 December 2016 (2016-12-15) * abstract * * paragraph [0071] * | 1-14 | |

TECHNICAL FIELDS
SEARCHED (IPC)

G16H

The present search report has been drawn up for all claims

| Place of search | Date of completion of the search | Examiner |
|---|---|---|
| Munich | 22 November 2019 | Schmitt, Constanze |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 38 2423

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2019

| Patent document cited in search report | | Publication date | Patent family member(s) | | Publication date |
|---|---|---|---|---|---|
| US 2012245481 | A1 | 27-09-2012 | NONE | | |
| US 2007250133 | A1 | 25-10-2007 | EP | 2010051 A2 | 07-01-2009 |
| | | | EP | 2012662 A2 | 14-01-2009 |
| | | | US | 2007250133 A1 | 25-10-2007 |
| | | | US | 2008046024 A1 | 21-02-2008 |
| | | | US | 2011263999 A1 | 27-10-2011 |
| | | | WO | 2007124190 A2 | 01-11-2007 |
| | | | WO | 2007124270 A2 | 01-11-2007 |
| WO 2016200952 | A1 | 15-12-2016 | US | 2018279939 A1 | 04-10-2018 |
| | | | WO | 2016200952 A1 | 15-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9326698 B2
- US 6678548 B1
- US 2016045127 A1
- US 2016287118 A1
- WO 2018005981 A1
- US 6594524 B2

### Non-patent literature cited in the description

- **ANDRZEJAK RG ; DAVID O ; GNATKOVSKY V ; WENDLING F ; BARTOLOMEI F ; FRANCIONE S et al.** Localization of epileptogenic zone on pre-surgical intracranial EEG recordings: toward a validation of quantitative signal analysis approaches. *Brain Topography,* 2015, vol. 28 (6), 832-837
- **BARTOLOMEI F ; CHAUVEL P ; WENDLING F.** Epileptogenicity of brain structures in human temporal lobe epilepsy: a quantified study from intracerebral EEG. *Brain,* 2008, vol. 131 (7), 1818-1830
- **DAVID O ; BLAUWBLOMME T ; JOB AS ; CHABARDÈS S ; HOFFMANN D ; MINOTTI L et al.** Imaging the seizure onset zone with stereo-electroencephalography. *Brain,* 2011, vol. 134 (10), 2898-2911
- **GEERTSEMA EE ; VISSER GH ; VELIS DN ; CLAUS SP ; ZIJLMANS M ; KALITZIN SN.** Automated seizure onset zone approximation based on nonharmonic high-frequency oscillations in human interictal intracranial eegs. *International Journal of Neural Systems,* 2015, vol. 25 (05), 1550015
- **GNATKOVSKY V ; FRANCIONE S ; CARDINALE F ; MAI R ; TASSI L ; LO RUSSO G et al.** Identification of reproducible ictal patterns based on quantified frequency analysis of intracranial EEG signals. *Epilepsia,* 2011, vol. 52 (3), 477-488
- **GNATKOVSKY V ; DE CURTIS M ; PASTORI C ; CARDINALE F ; LO RUSSO G ; MAI R et al.** Biomarkers of epileptogenic zone defined by quantified stereo-EEG analysis. *Epilepsia,* 2014, vol. 55 (2), 296-305
- **LIU S ; SHA Z ; SENCER A ; AYDOSELI A ; BEBEK N ; ABOSCH A et al.** Exploring the time-frequency content of high frequency oscillations for automated identification of seizure onset zone in epilepsy. *Journal of Neural Engineering,* 2016, vol. 13 (2), 026026
- A novel HFO-based method for unsupervised localization of the seizure onset zone in drug-resistant epilepsy. **MURPHY PM ; VON PATERNOS AJ ; SANTANIELLO S.** In: Engineering in Medicine and Biology Society (EMBC), 2017 39th Annual International Conference of the IEEE. IEEE, 2017, 1054-1057
- Inter-ictal seizure onset zone localization using unsupervised clustering and bayesian filtering. **VARATHARAJAH Y ; BERRY BM ; KALBARCZYK ZT ; BRINKMANN BH ; WORRELL GA ; IYER RK.** Neural Engineering (NER), 2017 8th International IEEE/EMBS Conference on. IEEE, 2017, 533-539
- **VILA-VIDAL M ; PRINCIPE A ; LEY M ; DECO G ; TAUSTE CAMPO A ; ROCAMORA R.** Detection of recurrent activation patterns across focal seizures: Application to seizure onset zone identification. *Clinical Neurophysiology,* 2017, vol. 128 (6), 977-985